# EUROPEAN PATENT APPLICATION

(11) **EP 2 105 147 A1**
(43) Date of publication of application: **30.09.2009**
(21) Application number: 09003921.5
(22) Date of filing: 18.03.2009
(51) Int. Cl.: A61L 2/025, B08B 3/12

(54) **Endoscope cleaner and cleaning method**

(30) Priority: 28.03.2008 JP 2008088693
(71) Applicant: FUJIFILM Corporation, Minato-ku Tokyo (JP)
(72) Inventor: Serizawa, Mitsuhiko, Kanagawa (JP); Iida, Takayuki, Kanagawa (JP)
(74) Representative: Klunker . Schmitt-Nilson . Hirsch

(57) **Abstract**

An endoscope cleaner includes a cleaning chamber (31) for containing an endoscope. A vibration plate (32) is disposed in a lower portion (31c) of the cleaning chamber. A basket portion (53) is disposed substantially at a center of the vibration plate, for containing small parts (21-23) removed from the endoscope. A nozzle opening (47a) supplies cleaning fluid (76) into the cleaning chamber through the basket portion. Plural ultrasonic vibrators (45) include at least one disposed under the basket portion, for vibrating the vibration plate. Furthermore, a drain port drains the cleaning fluid. A circulation line circulates the cleaning fluid from the cleaning chamber by use of the nozzle opening and the drain port. A disinfectant nozzle ejects disinfectant to disinfect the endoscope and the small parts. The basket portion has an upwards open shape, and prevents the small parts from scattering in vibration. The small parts are plural button parts or cap parts.

## Description

### BACKGROUND OF THE INVENTION

### 1. Field of the Invention

The present invention relates to an endoscope cleaner and cleaning method. More particularly, the present invention relates to an endoscope cleaner and cleaning method in which an endoscope and its small parts can be cleaned with high efficiency and in a cleaning chamber in a form without particular enlargement.

### 2. Description Related to the Prior Art

An endoscope is a medical instrument for use in examination of a patient's body and treatment. There is an insertion tube extending from a handle of the endoscope for entry in the body. The insertion tube is a flexible tube, and has a head assembly and various channels. The head assembly picks up an image of the body. One of the channels is a forceps channel for insertion of a treatment tool.

To clean and disinfect the endoscope efficiently, an endoscope cleaner is used. A cleaning chamber is formed in a metal body of the endoscope cleaner. The endoscope after use is contained in the cleaning chamber and automatically treated in a cleaning step and a disinfecting step. In the cleaning step, the cleaning chamber is supplied with water, detergent and the like, and removes dirt, pollution or the like from the surface and channels of the endoscope. In the disinfecting step, the endoscope is submerged in a bath of disinfectant in the cleaning chamber, to remove bacteria, virus or other microbe remaining even after the cleaning step, or to remove pathogenicity of such microbes.

JP-A 6-007290 (corresponding to JP-B 2660137) discloses the endoscope cleaner of ultrasonic type to clean the endoscope. There is a vibration plate disposed in a lower surface of the cleaning chamber in the endoscope cleaner for the ultrasonic cleaning. Plural ultrasonic vibrators are associated with a lower surface of the vibration plate. Cleaning fluid containing water and detergent is contained in the cleaning chamber. In the cleaning step, the endoscope is submerged in a bath of the cleaning fluid. The ultrasonic vibrators are driven to vibrate the cleaning fluid by means of the vibration plate to clean the endoscope ultrasonically.

As the endoscope is contained in the cleaning chamber with the insertion tube flexed in a looped form, there occurs an open space at the center of the cleaning chamber. In the endoscope cleaner of JP-A 6-007290 (corresponding to JP-B 2660137), a chamber post portion of a rod shape is disposed at the center of the cleaning chamber to define a ring shape of an inner space of the cleaning chamber for the purpose of reducing the total of fluid charged in the cleaning chamber. The insertion tube of the endoscope is set about the chamber post portion in the circularly flexed shape. Thus, the use of the chamber post portion is effective in economizing the supply of the cleaning fluid. Note that plural ultrasonic vibrators are arranged in a ring shape because the cleaning chamber has the ring shape.

Furthermore, a mesh basket is used in the cleaning chamber for containing small parts which may be an air/water supply button, a suction button, and a forceps channel cap removed from the handle of the endoscope. If the center of the cleaning chamber has the chamber post portion as suggested in JP-A 6-007290 (corresponding to JP-B 2660137), the center area of the cleaning chamber cannot be used for disposing the mesh basket. Otherwise, the mesh basket needs be disposed in the space of the cleaning chamber for containing the insertion tube of the endoscope. The size of the cleaning chamber will be considerably great to increase the total of the cleaning fluid for use.

Should the small parts be cleaned in a separate manner from the endoscope, the efficiency in the cleaning will be considerably low.

The air/water supply button and suction button, which operate as valves for opening and closing the air/water supply channel and suction channel, specifically have recesses, projections, grooves and the like on the surface. It is great concern to raise efficiency in cleaning the small parts where dirt or pollution is considerably difficult to remove.

### SUMMARY OF THE INVENTION

In view of the foregoing problems, an object of the present invention is to provide an endoscope cleaner and cleaning method in which an endoscope and its small parts can be cleaned with high efficiency and in a cleaning chamber in a form without particular enlargement.

In order to achieve the above and other objects and advantages of this invention, an endoscope cleaner includes a cleaning chamber for containing an endoscope. A vibration plate is disposed in a lower portion of the cleaning chamber. A basket portion is disposed substantially at a center of the vibration plate, for containing a small part removed from the endoscope. A nozzle opening supplies cleaning fluid into the cleaning chamber through the basket portion. Plural ultrasonic vibrators include at least one disposed under the basket portion, for vibrating the vibration plate.

The nozzle opening and the at least one ultrasonic vibrator under the basket portion are disposed to protrude at least partially in a projected portion defined by projecting the basket portion down toward the vibration plate.

The basket portion is constituted by mesh material.

The plural ultrasonic vibrators are arranged radially on circles which are concentric on a plane parallel with a surface of the vibration plate, and at least one of the ultrasonic vibrators is disposed under the basket portion and positioned on an innermost one of the circles.

The plural ultrasonic vibrators are arranged in a spiral form starting from a point under the basket portion on a plane parallel with a surface of the vibration plate.

Furthermore, a disinfectant supply line supplies the cleaning chamber with disinfectant to disinfect the endoscope and the small part.

Furthermore, a supply line supplies the cleaning chamber with the cleaning fluid. A drain port is formed in the cleaning chamber, for draining the cleaning fluid. A circulation line circulates the cleaning fluid from the cleaning chamber through the nozzle opening and the drain port.

Furthermore, a water supply line supplies the cleaning chamber with water after cleaning the endoscope and the small part and before disinfection, to rinse the cleaning chamber. The circulation line circulates the disinfectant after the disinfectant supply line operates, and circulates the water after the water supply line operates.

The basket portion has an upwards open shape, and prevents the small part from scattering in vibration.

The basket portion has a width increasing upwards.

The small part is constituted by plural button parts or cap parts.

The endoscope includes a handle portion. An insertion tube is disposed to extend from the handle portion. A cable is disposed to extend from the handle portion. A peripheral portion of the cleaning chamber is assigned with the handle portion. The vibration plate supports the insertion tube and the cable upwards.

In one aspect of the invention, an endoscope cleaning method of cleaning an endoscope is provided, and includes a step of placing the endoscope in a cleaning chamber and a small part thereof in a basket portion which is disposed substantially at a center of the cleaning chamber. Cleaning fluid is supplied through at least one nozzle opening into the cleaning chamber for circulation cleaning of the endoscope and the small part, the nozzle opening being disposed under the basket portion. The vibration plate disposed in a lower portion of the cleaning chamber is vibrated by use of an ultrasonic vibrator disposed under the basket portion at least partially.

Furthermore, after the cleaning step, the cleaning chamber is rinsed with water. After the rinsing step, the endoscope and the small part are disinfected with disinfectant.

Furthermore, after the disinfecting step, the cleaning chamber is rinsed with water. The endoscope and the small part are dried with evaporative gas.

The circulation cleaning step includes draining the cleaning fluid from the cleaning chamber through a drain port. The cleaning fluid is circulated from the cleaning chamber by use of the nozzle opening and the drain port.

Accordingly, the endoscope and its small parts can be cleaned with high efficiency and in a cleaning chamber in a form without particular enlargement.

### BRIEF DESCRIPTION OF THE DRAWINGS

The above objects and advantages of the present invention will become more apparent from the following detailed description when read in connection with the accompanying drawings, in which:
Fig. 1 is a plan partially broken illustrating construction of an endoscope;
Fig. 2 is a perspective view illustrating an endoscope cleaner;
Fig. 3 is a plan illustrating a cleaning chamber;
Fig. 4 is a cross section illustrating the cleaning chamber;
Fig. 5 is an explanatory view in plan illustrating arrangement of ultrasonic vibrators relative to a vibration plate;
Fig. 6 is a flow diagram illustrating a flow system in connection with the endoscope cleaner;
Fig. 7 is a block diagram illustrating the endoscope cleaner;
Fig. 8 is a flow chart illustrating a process of the cleaning and disinfection;
Fig. 9 is an explanatory view in plan illustrating another preferred arrangement of ultrasonic vibrators.

### DETAILED DESCRIPTION OF THE PREFERRED EMBODIMENT(S) OF THE PRESENT INVENTION

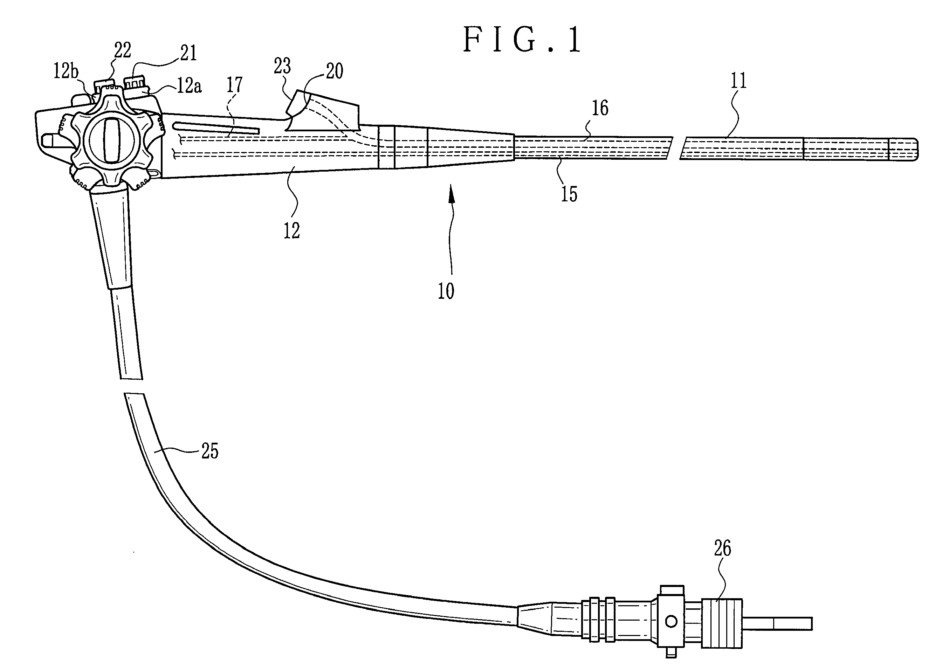

In Fig. 1, an endoscope 10 of an example is cleaned in an endoscope cleaner of the present invention. The endoscope 10 includes an insertion tube 11 and a handle portion 12. The insertion tube 11 is swallowed orally in a body of a patient. The handle portion 12 is manually operable by a doctor for control of the insertion tube 11.

The insertion tube 11 has a form of a circle when viewed in a cross section, and has flexibility. A distal end of the insertion tube 11 has an illuminator and an imaging window (not shown). The illuminator applies light to an object in the body. The imaging window receives image light for imaging. An air/water supply channel 15 and a forceps channel 16 are formed through the insertion tube 11, and have end openings at its distal end. A suction channel 17 extends from an intermediate point of the forceps channel 16.

The handle portion 12 has a forceps opening 20, an air/water supply button 21 and a suction button 22. A forceps channel cap 23 is fitted on the forceps opening 20, and is removed at the time of the use. Button openings 12a and 12b are formed in the handle portion 12, and receive the air/water supply button 21 and the suction button 22 in a depressible form and in a removable manner. To clean the endoscope 10, the forceps channel cap 23, the air/water supply button 21 and the suction button 22 are removed from the handle portion 12.

A universal cable 25 extends from the handle portion 12. A connector 26 is provided at an end of the universal cable 25. Various elements are contained in the universal cable 25 and the connector 26, including the air/water supply channel 15, the suction channel 17, and wires for an illuminator and image pickup device. Contacts are included in the connector 26 for connection of the wires with a light source device, a video processor and the like. A watertight cap (not shown) is fitted on the connector 26 to protect the contacts from fluid at the time of cleaning the endoscope 10.

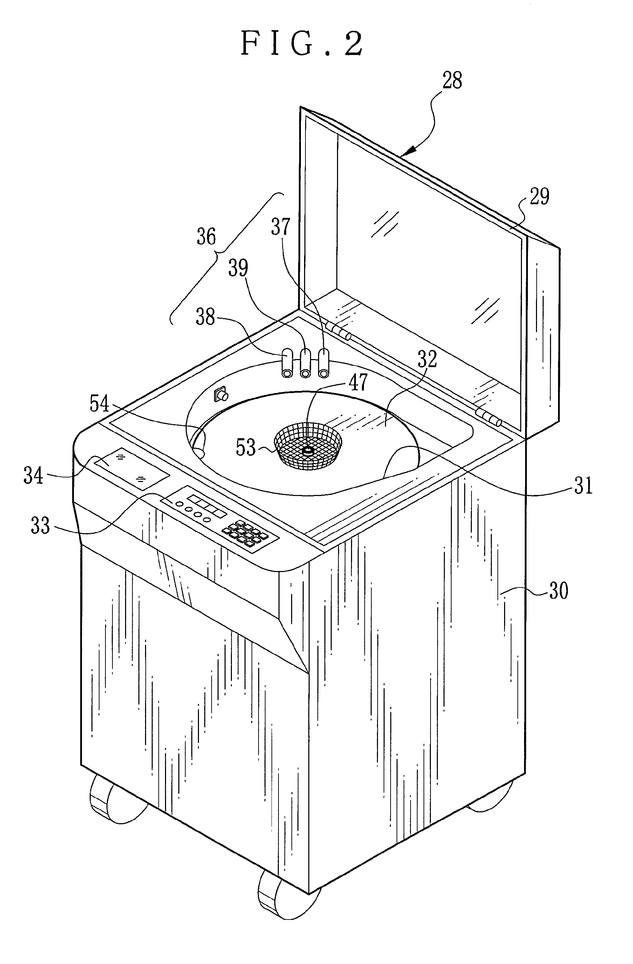

In Fig. 2, an endoscope cleaner 28 cleans the endoscope 10 after the use. The endoscope cleaner 28 has a cleaner body 30. A cleaning chamber 31 is formed in an upper side of the cleaner body 30 for containing the endoscope 10 after the use. The cleaning chamber 31 is defined by a fluid tank, which has an open top, and is constituted by metal plates with high resistance to heat and resistance to corrosion, for example stainless steel. An openable top cover 29 covers the upper side of the cleaning chamber 31 in an openable manner.

An input panel 33 and a display panel 34 are disposed on the top side of the cleaner body 30. The input panel 33 includes various buttons for inputting information of cleaning and disinfection of the endoscope 10, start and stop of cleaning and disinfection of the endoscope 10 and the like. An example of the display panel 34 is a liquid crystal display (LCD) which displays information of various menus for setting, remaining time of each of processes, warning message upon occurrence of a problem, and the like.

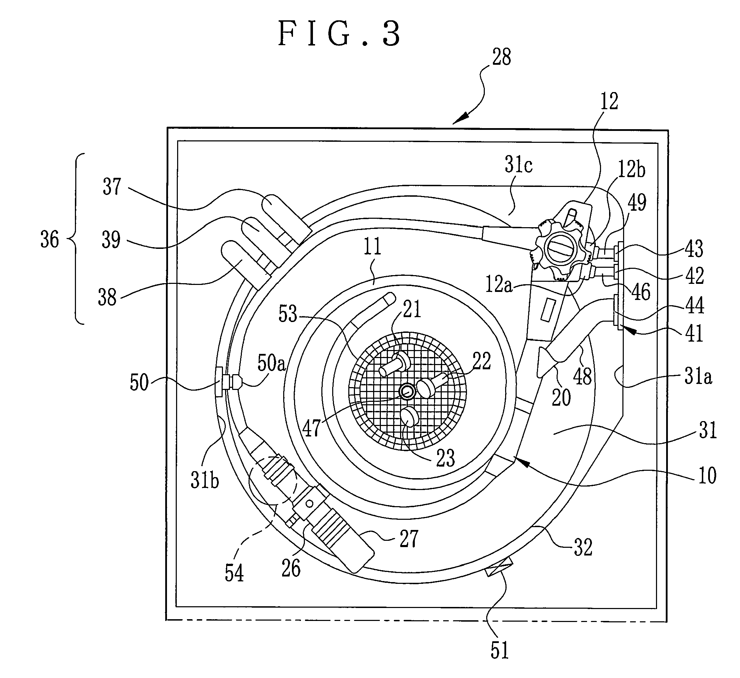

In Fig. 3, a supply port 36 is formed in the upper side of the cleaner body 30 for supply of fluid to the cleaning chamber 31. There are a water nozzle 37, a detergent nozzle 38 or washing agent nozzle, and a disinfectant nozzle 39 in the supply port 36. The water nozzle 37 ejects water down to the cleaning chamber 31. The detergent nozzle 38 ejects detergent down to the cleaning chamber 31, for example, enzymatic liquid detergent. The disinfectant nozzle 39 ejects disinfectant down to the cleaning chamber 31, for example, glutalaldehyde, peracetic acid, ortho phthalaldehyde, and the like.

A channel washing port 41 is formed in an inner lateral surface 31a of the cleaning chamber 31. The channel washing port 41 is used to clean and disinfect the air/water supply channel 15, the forceps channel 16 and the suction channel 17 in the endoscope 10. There are an air/water channel coupler 42, a suction channel coupler 43 and a forceps channel coupler 44 in the channel washing port 41.

Tubes 46, 48 and 49 of a flexible form extend from the endoscope 10 in the cleaning chamber 31. The tube 46 connects the button opening 12a to the air/water channel coupler 42. The tube 48 connects the forceps opening 20 to the forceps channel coupler 44. The tube 49 connects the button opening 12b to the suction channel coupler 43. The channel couplers 42-44 supply the air/water supply channel 15, the forceps channel 16 and the suction channel 17 with fluid and gas, specifically, water, cleaning fluid, disinfectant, alcohol, and compressed air.

An air leak test port 50 is formed in an inner peripheral surface 31b of the cleaning chamber 31, and used for the test of air leak of the endoscope 10. A tube coupler 50a is provided on the air leak test port 50 for supplying compressed air. One end of a flexible tube is connected with the tube coupler 50a. A second end of the flexible tube is connected with an orifice formed in the connector 26 for the air leak test. A watertight cable cap 27 is fitted on the connector 26 and prevents entry of water. A liquid surface sensor 51 is disposed on the inner peripheral surface 31b. The liquid surface sensor 51 detects a bath surface of the fluid contained in the cleaning chamber 31.

A vibration plate 32 of a disk shape is disposed on a lower surface 31c of the cleaning chamber 31. A mesh basket portion 53 is attached on an upper surface of the vibration plate 32. An example of the mesh basket portion 53 has a shape of a cup with an open top in a circular form, and is formed in a mesh pattern by knitting material with high resistance to erosion, for example stainless steel. The mesh basket portion 53 is assigned with the air/water supply button 21, the suction button 22, the forceps channel cap 23 and the like as small parts removed from the handle portion 12 of the endoscope 10. The small parts are cleaned together with the endoscope 10 within the mesh basket portion 53. Also, a drain port 54 is formed in the lower surface 31c. The drain port 54 drains used water, cleaning fluid and disinfectant from the cleaning chamber 31.

By way of the mesh basket portion 53, it is possible to use any structure for preventing the small parts from scattering, and for freely passing fluid, for example, net, filter, holder, rack, receptacle and various other elements which can be used in parts washers of well-known techniques.

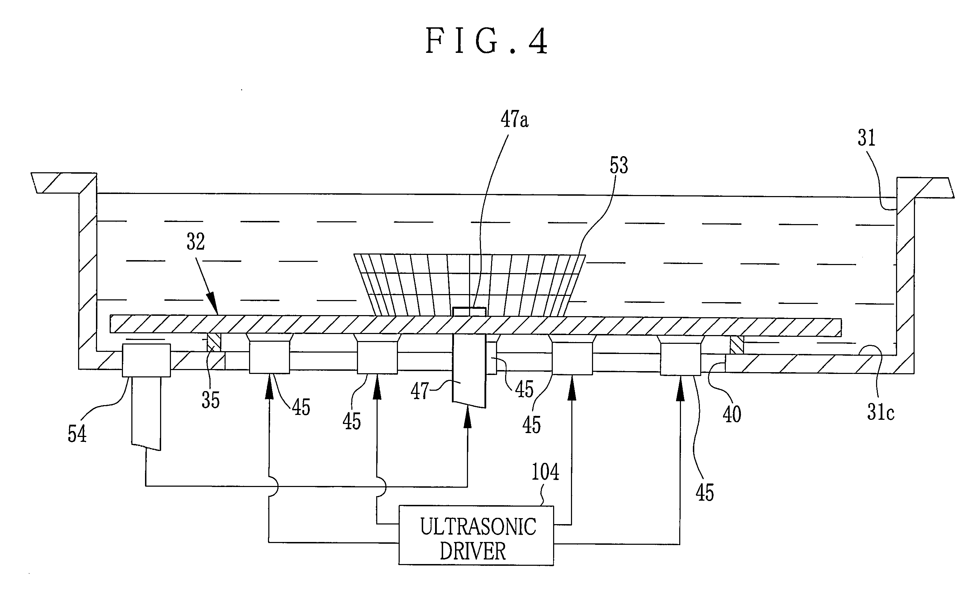

The vibration plate 32 is formed from stainless steel or other material having high resistance to erosion. In Fig. 4, the vibration plate 32 is positioned higher than the lower surface 31c with a small space. A rubber ring 35 is positioned between the vibration plate 32 and the lower surface 31c for the purpose of packing in a watertight manner and protection from vibration. A circular opening 40 is formed in the lower surface 31c and located inside the rubber ring 35. Ultrasonic vibrators 45 are secured to a lower surface of the vibration plate 32 in a space defined by the circular opening 40. An ultrasonic driver 104 drives the ultrasonic vibrators 45 to vibrate the vibration plate 32.

A fluid nozzle 47 has a nozzle opening 47a. The cleaning fluid, disinfectant 81 or water is ejected by the nozzle opening 47a for supply to the cleaning chamber 31 as the fluid of circulation. The fluid nozzle 47 extends through the circular opening 40 toward the cleaning chamber 31 up from a position under the vibration plate 32. Its end having the nozzle opening 47a projects from the vibration plate 32 in the cleaning chamber 31 at a small height by penetration through the center of the vibration plate 32. As the mesh basket portion 53 is disposed in the center of the vibration plate 32, the end of the fluid nozzle 47 is located in the mesh basket portion 53. Fluid ejected by the nozzle opening 47a becomes charged in the cleaning chamber 31 by passage through the mesh basket portion 53.

The nozzle opening 47a is positioned substantially at the center of the mesh basket portion 53. Thus, streams of fluid from the nozzle opening 47a reach the inside of the mesh basket portion 53. Impact energy of the fluid can be applied to the surface of small parts in the mesh basket portion 53, effectively to remove dirt from grooves, uneven patterns with recesses, or the like on the surface of the small parts.

In the present embodiment, the position of the nozzle opening 47a in the horizontal direction is defined at the center of the mesh basket portion 53. However, the position of the nozzle opening 47a in the horizontal direction may be eccentric with the mesh basket portion 53, to supply the fluid from the nozzle opening 47a to the cleaning chamber 31 through the mesh basket portion 53.

However, should the distance of the nozzle opening 47a be too great from the mesh basket portion 53, impact energy of the fluid ejected by the nozzle opening 47a is difficult to transmit to the small parts. Thus, it is preferable to determine the position of the nozzle opening 47a in the horizontal direction within a contour of a projected image defined by projecting the mesh basket portion 53 vertically down on a surface of the vibration plate 32.

The mesh basket portion 53 has a diameter increasing in an upward direction. The diameter is the maximum at its upper end. The inside of the contour of the projected image is herein referred to for including the inside of the contour of the projected image of the upper opening of the mesh basket portion 53 in addition of its lower region. Also, the nozzle opening 47a may not be entirely located inside the contour. At least one portion of the nozzle opening 47a may appear within the contour. Furthermore, two or more nozzle openings 47a may be provided in the contour according to the present invention.

The nozzle opening 47a preferably has such a small height that its top is near to the lower surface of the mesh basket portion 53, because the nozzle opening 47a operates to direct a flow of fluid to the surface of the small parts in the mesh basket portion 53. Also, the nozzle opening 47a may be flush with or lower than a lower surface of the mesh basket portion 53.

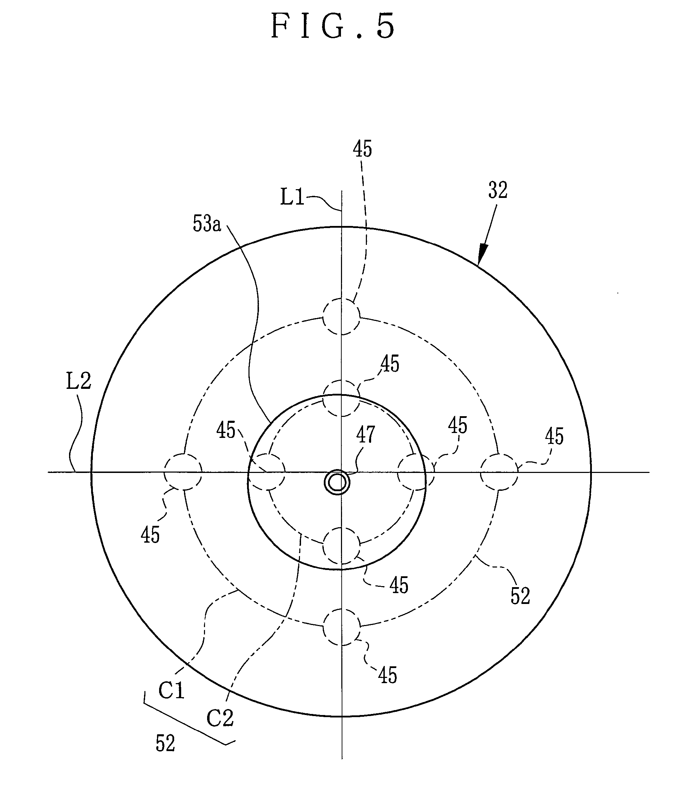

In Fig. 5, the ultrasonic vibrators 45 are arranged on concentric circles 52 on a plane parallel with the vibration plate 32, and in radial directions. The concentric circles 52 are substantially concentric with the vibration plate 32. The concentric circles 52 include circles C1 and C2 with different diameters. The ultrasonic vibrators 45 are arranged at points of intersection between the circles C1 and C2 and lines L1 and L2 which cross each other vertically at the center of the concentric circles 52. In short, the ultrasonic vibrators 45 are arranged on the lines L1 and L2 extending in the radial directions.

The ultrasonic vibrators 45 are eight vibrators. Four of those are positioned at points of intersection of the circle C2 and the lines L1 and L2. Other four of the ultrasonic vibrators 45 are positioned at points of intersection of the outer circle C1 and the lines L1 and L2. Thus, the ultrasonic vibrators 45 are arranged at a regular interval on the surface of the vibration plate 32. In the vicinity of the ultrasonic vibrators 45, amplitude of the vibration plate 32 is relatively high in the vibration. However, the regular arrangement of the ultrasonic vibrators 45 is effective in ultrasonic cleaning regularly to the entirety of the fluid in the cleaning chamber 31.

The four of the ultrasonic vibrators 45 at the points of intersection of the circle C2 and the lines L1 and L2 are arranged to project in a contour 53a of the mesh basket portion 53 at least partially. See Fig. 5. The amplitude of the vibration plate 32 is comparatively large in the vicinity of the ultrasonic vibrators 45. Arrangement of the ultrasonic vibrators 45 under the mesh basket portion 53 is effective in raising performance in cleaning the small parts.

In JP-A 6-007290 (corresponding to JP-B 2660137), plural ultrasonic vibrators are arranged in a circular form on the inside of the circular cleaning chamber. If the ultrasonic vibrators are disposed under the mesh basket portion 53 in the circular arrangement, the diameter of the circle will be excessively small, to cause unwanted concentrated arrangement of the ultrasonic vibrators at the center of the vibration plate 32. However, the ultrasonic vibrators 45 are arranged in the radial directions of the concentric circles 52. Four of the ultrasonic vibrators 45 on the inner circle are disposed under the mesh basket portion 53. Four of the ultrasonic vibrators 45 on the outer circle are near to the periphery of the vibration plate 32. This is effective in solving the problem of the unwanted concentrated arrangement at the center of the vibration plate 32.

In the embodiment, the four of the ultrasonic vibrators 45 are disposed to project into the area of the contour 53a. However, at least one of the ultrasonic vibrators 45 can be disposed to project into the area of the contour 53a. One of the ultrasonic vibrators 45 may not entirely project into the area of the contour 53a. At least one portion of one of the ultrasonic vibrators 45 can project into the area of the contour 53a.

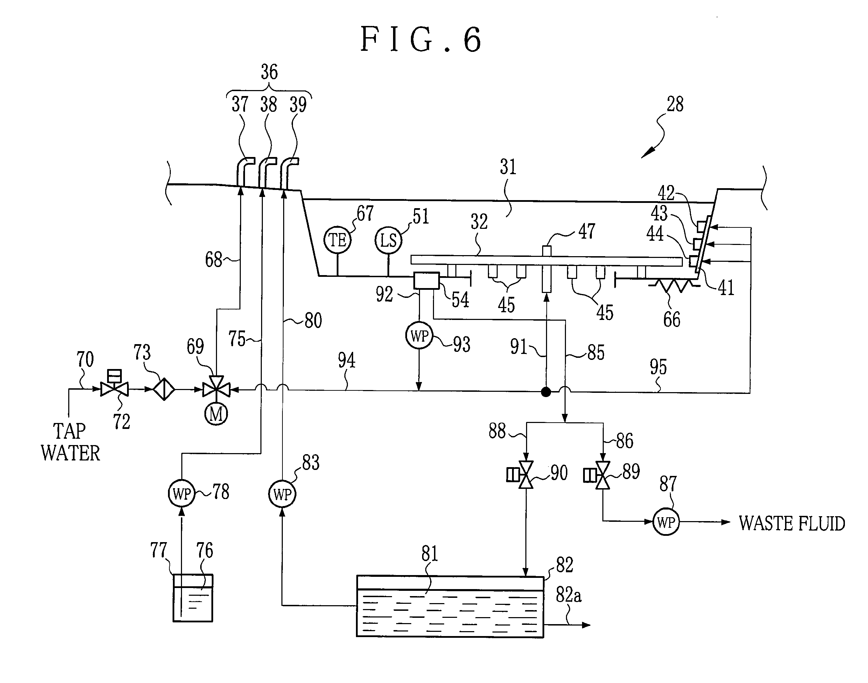

In Fig. 6, a flow system in the cleaner body 30 is illustrated. A rubber heater 66 is disposed on the lower surface of the cleaning chamber 31. The rubber heater 66 applies heat to cleaning fluid or disinfectant stored in the cleaning chamber 31. A temperature sensor 67 (TE) is disposed in the cleaning chamber 31, and measures the bath temperature of the cleaning fluid or disinfectant.

A flow line 68 is connected with the water nozzle 37 for flow of water, cleaning fluid and disinfectant. There is an electric three-way valve 69 with one valve opening where a second end of the flow line 68 is connected. A second valve opening of the three-way valve 69 is connected with a water supply line 70. The water supply line 70 extends externally to appear outside the cleaner body 30, and is connected with a tap water source.

The water supply line 70 includes an electromagnetic valve 72 and a water filter 73. The electromagnetic valve 72 is nearer to the tap water source, and changes over flow and stop of water in the water supply line 70. The water filter 73 captures foreign material, microbes and other unwanted particles in the water. The three-way valve 69 connects the flow line 68 with the water supply line 70 in the supply of water to the cleaning chamber 31.

A supply line 75 for detergent or washing agent extends to the detergent nozzle 38. A second end of the supply line 75 is connected with a detergent tank or washing agent tank 77. Detergent or washing agent 76 is contained in the detergent tank 77. A fluid pump 78 is provided in the supply line 75. The detergent 76 in the detergent tank 77 is drawn by the fluid pump 78 and ejected through the detergent nozzle 38.

A disinfectant supply line 80 extends to the disinfectant nozzle 39. A disinfectant tank 82 stores the disinfectant 81, and is connected with a proximal end of the disinfectant supply line 80. A fluid pump 83 is provided in the disinfectant supply line 80. The fluid pump 83 draws the disinfectant 81 from the disinfectant tank 82, and causes the disinfectant nozzle 39 to eject the disinfectant 81. A discharge line 82a extends from the disinfectant tank 82 to discharge the disinfectant 81 finally as waste.

A drain line 85 is connected with the drain port 54. A first drain line 86 drains the cleaning fluid and water after cleaning the endoscope 10 to the outside of the cleaner body 30 by use of a fluid pump 87. A second drain line 88 returns the disinfectant 81 to the disinfectant tank 82 after use for disinfecting the endoscope 10. The disinfectant 81 is repeatedly used by return to the disinfectant tank 82, as its effect of disinfection can maintain even in a plurality of times of the use for disinfection. Electromagnetic valves 89 and 90 are operable for changing over the first drain line 86 and the second drain line 88.

A circulation line 92 is also connected with the drain port 54 for circulating cleaning fluid, the disinfectant 81 and the water in the cleaning chamber 31. A fluid pump 93 is provided in the circulation line 92 for drawing fluid from the cleaning chamber 31. Branches of the circulation line 92 extend from a downstream end of the circulation line 92 at the fluid pump 93, including a first circulation line 94, a second circulation line 95 and a mesh basket circulation line 91. The first circulation line 94 is connected with the three-way valve 69. The three-way valve 69 changes over for communication of the flow line 68 and the first circulation line 94 to circulate the cleaning fluid, the disinfectant 81 and the water in the cleaning chamber 31.

The second circulation line 95 is connected with the air/water channel coupler 42, the suction channel coupler 43 and the forceps channel coupler 44 of the channel washing port 41. The cleaning fluid, the disinfectant 81 and the water in the second circulation line 95 flow through the channel couplers 42-44 of the channel washing port 41 and the tubes 46-48, to clean and disinfect the air/water supply channel 15, the forceps channel 16 and the suction channel 17.

The mesh basket circulation line 91 is connected with the fluid nozzle 47. The cleaning fluid, the disinfectant 81 and the water through the mesh basket circulation line 91 are ejected by the fluid nozzle 47, and clean and disinfect the small parts contained in the mesh basket portion 53 with a stream or a fluid bath.

To the channel washing port 41 are connected the second circulation line 95, an air supply line, and alcohol supply line. The air supply line supplies air to the inside of the channels to remove water droplets. The alcohol supply line supplies alcohol to the channels for drying. Note that the air supply line and alcohol supply line are not depicted in Fig. 6 for the purpose of simplicity.

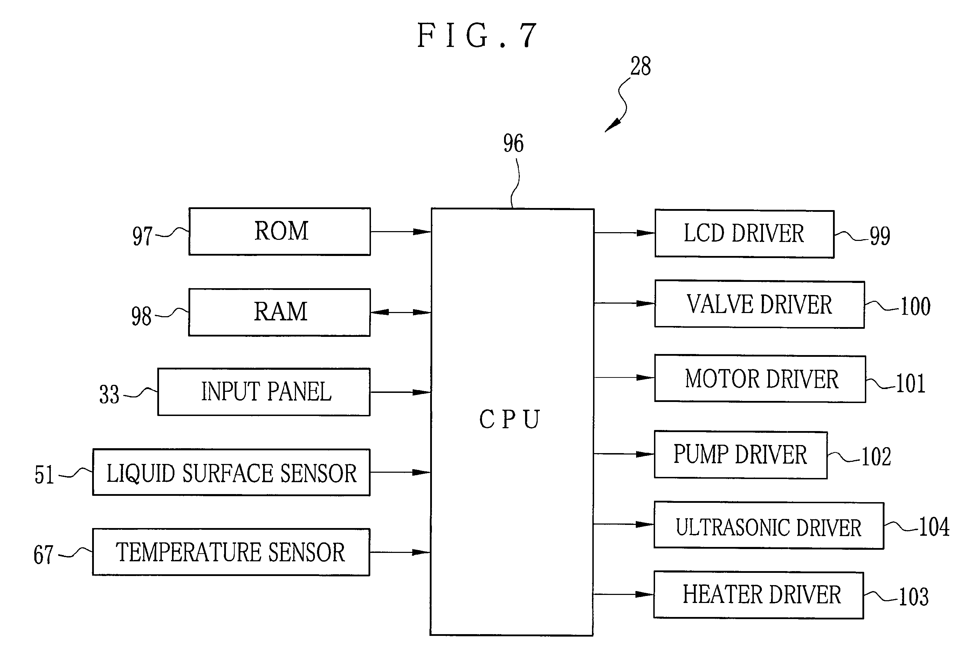

In Fig. 7, the endoscope cleaner 28 includes a CPU 96, a ROM 97 and a RAM 98. The ROM 97 stores a control program and various data. The RAM 98 is a working memory for running the control program read from the ROM 97. Various elements are connected with the CPU 96, including the liquid surface sensor 51, the temperature sensor 67, an LCD driver 99, a valve driver 100, and the ultrasonic driver 104. The LCD driver 99 drives the display panel 34. The valve driver 100 drives respective electromagnetic valves. The ultrasonic driver 104 drives the ultrasonic vibrators 45. Also, a motor driver 101, a pump driver 102 and a heater driver 103 are connected with the CPU 96. The motor driver 101 drives the three-way valve 69. The pump driver 102 drives the fluid pumps. The heater driver 103 drives the rubber heater 66.

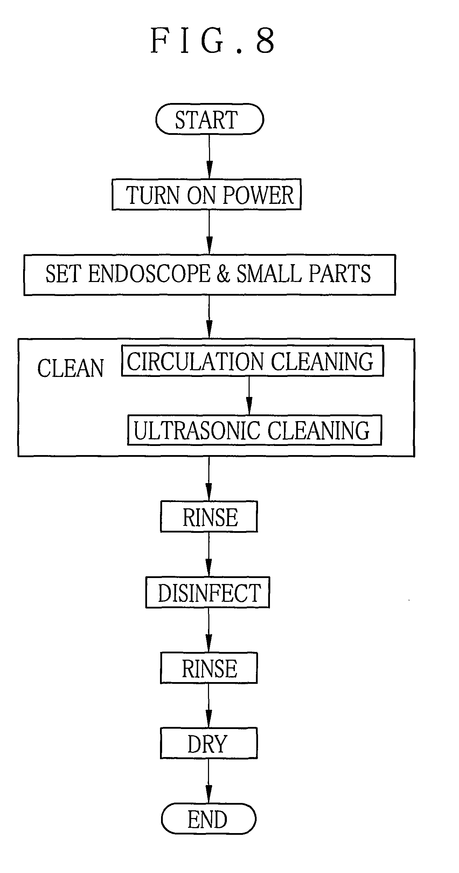

Cleaning and disinfection of the endoscope 10 in the endoscope cleaner 28 are described by referring to Fig. 8.

At first, small parts are removed from the handle portion 12 of the endoscope 10, including the air/water supply button 21, the suction button 22 and the forceps channel cap 23. Then the input panel 33 on the endoscope cleaner 28 is operated manually to turn on the power source. The top cover 29 is opened, to set the endoscope 10. The small parts are placed in the mesh basket portion 53. The button openings 12a and 12b of the handle portion 12 are opposed to the channel washing port 41 in the cleaning chamber 31. The insertion tube 11 is flexed in a ring shape and placed on the vibration plate 32 and around the mesh basket portion 53. The universal cable 25 with the connector 26 is flexed and placed near to the air leak test port 50. The watertight cable cap 27 is fitted on the connector 26.

The tubes 46, 48 and 49 are connected to respectively the air/water channel coupler 42, the forceps channel coupler 44 and the suction channel coupler 43 of the channel washing port 41. Also, the tubes 46, 48 and 49 are connected to the button opening 12a, the forceps opening 20 and the button opening 12b in the endoscope 10.

After the endoscope 10 is placed in the cleaning chamber 31, the top cover 29 is closed. A full automatic mode is selected for starting, in which the cleaning and disinfection are carried out fully. Cleaning of the endoscope 10 is started.

In the cleaning and disinfection, there are five successive steps, including cleaning, rinsing, disinfection, rinsing and drying. In the cleaning step, at first the cleaning chamber 31 is supplied with water. The valve driver 100 is controlled by the CPU 96 to open the electromagnetic valve 72. The motor driver 101 is controlled to change over the three-way valve 69 for communication between the flow line 68 and the water supply line 70. Water supplied through the water supply line 70 by water pressure of the tap water source is purified by the water filter 73. The water from the water supply line 70 is drawn in the flow line 68, and ejected by the water nozzle 37 into the cleaning chamber 31.

Then the cleaning chamber 31 is charged with the detergent 76. At the same time as the start of the supply to the cleaning chamber 31, the CPU 96 drives the fluid pump 78 and causes the detergent nozzle 38 to eject the detergent 76 of a predetermined amount into the cleaning chamber 31. Thus, cleaning fluid is created in the cleaning chamber 31 by mixing the water and the detergent 76. An amount of the cleaning fluid in the cleaning chamber 31 is detected by the liquid surface sensor 51. When a bath surface of the cleaning fluid reaches the target position, then the CPU 96 closes the electromagnetic valve 72 to stop the supply of water.

After the supply in the cleaning chamber 31, the cleaning fluid is heated to and maintained at a predetermined temperature. The heater driver 103 is controlled by the CPU 96 to drive the rubber heater 66. Heat from the rubber heater 66 is transmitted to the cleaning fluid by the cleaning chamber 31. Its bath temperature is detected by the temperature sensor 67. The rubber heater 66 is controlled by the CPU 96 for a constant level of the bath temperature.

Then the circulation cleaning and ultrasonic cleaning are carried out successively. The CPU 96 changes over the three-way valve 69 to communicate between the flow line 68 and the first circulation line 94, and drives the fluid pump 93. The cleaning fluid drained in the drain port 54 flows through the circulation line 92, the first circulation line 94 and the flow line 68, and ejected by the water nozzle 37 toward the endoscope 10. Pollution, dirt or the like deposited on the surface of the endoscope 10 is cleaned away with impact energy of the stream of the cleaning fluid, and vortex in the circulation. A gradient of the density of the detergent is set smaller by the circulation of the cleaning fluid, to obtain effect of cleaning in the entirety of the cleaning chamber 31.

Cleaning fluid drawn through the circulation line 92 flows in the second circulation line 95, the channel washing port 41 and the like and supplied to the endoscope 10. The cleaning fluid in the endoscope 10 cleans the air/water supply channel 15, the forceps channel 16 and the suction channel 17.

The cleaning fluid drawn in the circulation line 92 flows through the mesh basket circulation line 91 and the fluid nozzle 47 toward the mesh basket portion 53. The cleaning fluid in the mesh basket portion 53 cleans the small parts. Dirt on the small parts, which would not be removed according to the known techniques, is washed away by impact energy of the cleaning fluid ejected by the fluid nozzle 47 and its vortex flow at the time of circulation.

When a predetermined time elapses, the CPU 96 stops the fluid pump 93 to stop the circulation cleaning. After this, the CPU 96 causes the ultrasonic driver 104 to drive the ultrasonic vibrators 45 for a predetermined time.

The ultrasonic vibrators 45 cause the vibration plate 32 to vibrate. The vibration plate 32 emits ultrasonic waves in the cleaning fluid. Thus, the small parts in the mesh basket portion 53 and the endoscope 10 set about the same can be cleaned reliably. At least one of the ultrasonic vibrators 45 is disposed under the mesh basket portion 53, so that dirt on a portion of a complicated shape can be removed easily.

After the ultrasonic cleaning, the CPU 96 opens the electromagnetic valve 89 to drive the fluid pump 87. Cleaning fluid used in the cleaning is drained through the drain port 54, the drain line 85 and the first drain line 86, and discharged from the cleaner body 30 externally. Note that the fluid pump 93 is stopped after complete discharge of the cleaning fluid, for the purpose of clearing the cleaning fluid from the circulation line 92.

Then the endoscope 10 is rinsed with water to remove the cleaning fluid from the endoscope 10. For this purpose, the CPU 96 causes supply of water of a predetermined amount in the cleaning chamber 31. The water is circulated to remove cleaning fluid from the surface of the endoscope 10 and inner surfaces of the channels. The supply and the discharge of the water are the same as those at the time of cleaning.

The CPU 96 drives the fluid pump 83 to supply the cleaning chamber 31 with the disinfectant 81. The liquid surface sensor 51 detects a bath surface of the disinfectant 81 in the cleaning chamber 31. The CPU 96 receives and evaluates a detection signal from the liquid surface sensor 51, and stops the fluid pump 83 when the bath surface of the disinfectant 81 comes to the target position.

The CPU 96 drives the rubber heater 66 to apply heat to the disinfectant 81 to a target temperature by utilizing the temperature sensor 67. Then the CPU 96 drives the fluid pump 93 to circulate the disinfectant of the cleaning chamber 31 in the channels and the mesh basket portion 53 in the manner similar to the circulation cleaning. The surface of the endoscope 10 and its channels are submerged in and disinfected by the bath of the disinfectant 81. Also, the small parts in the mesh basket portion 53 are disinfected by the bath of the disinfectant 81. When a predetermined time elapses, the CPU 96 opens the electromagnetic valve 90 to draw the disinfectant 81 back to the disinfectant tank 82.

Rinsing follows the disinfection for the purpose of removing the disinfectant 81 from the endoscope 10. After the rinsing, the CPU 96 causes a blower to introduce air to the inside of channels in the endoscope 10 and small parts in the mesh basket portion 53 for removing water droplets. Then alcohol is supplied to the channels of the endoscope 10 for drying. Then the top cover 29 is opened upon completion of the cleaning and disinfection. The endoscope 10 is unloaded from the cleaning chamber 31.

In the embodiment, the arrangement of the ultrasonic vibrators 45 is eccentric as viewed from the mesh basket portion 53. See Fig. 5. However, it is possible to arrange the group of the ultrasonic vibrators 45 concentrically with the form of the mesh basket portion 53.

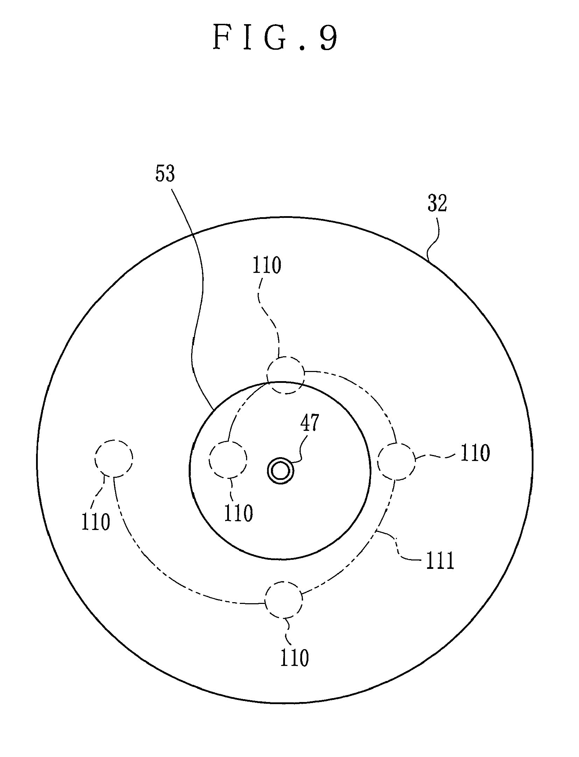

In the above embodiment, the ultrasonic vibrators 45 are arranged on the concentric circles. In contrast, in Fig. 9, ultrasonic vibrators 110 are arranged on a curve of a spiral which starts to extend from a position under the mesh basket portion 53 on a surface parallel to the form of the vibration plate 32.

A radius of the spiral form increases gradually toward the periphery of the vibration plate 32. One of the ultrasonic vibrators 45 at the start point of the spiral form is disposed under the mesh basket portion 53. Remaining ones of the ultrasonic vibrators 45 are arranged discretely toward the periphery of the vibration plate 32. Thus, the ultrasonic vibrators 45 are arranged at a regular interval on the surface of the vibration plate 32. Thus, speed of cleaning the small parts in the mesh basket portion 53 can be high owing to prevention of concentrated arrangement of the ultrasonic vibrators 45 in the surface of the vibration plate 32. This is a specific effect of the invention distinct from the arrangement suggested in JP-A 6-007290 (corresponding to JP-B 2660137).

Also, the spiral arrangement is suitable for the form of containing the insertion tube 11 around the mesh basket portion 53. Ultrasonic wave can be applied to the insertion tube 11 efficiently, to raise performance of cleaning.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An endoscope cleaner comprising:
a cleaning chamber (31) for containing an endoscope (10);
a vibration plate (32) disposed in a lower portion (31c) of said cleaning chamber;
a basket portion (53), disposed substantially at a center of said vibration plate, for containing a small part (21-23) removed from said endoscope;
a nozzle opening (47a) for supplying cleaning fluid (76) into said cleaning chamber through said basket portion; and
plural ultrasonic vibrators (45), including at least one disposed under said basket portion, for vibrating said vibration plate.

2. An endoscope cleaner as defined in claim 1, wherein said nozzle opening and said at least one ultrasonic vibrator under said basket portion are disposed to protrude at least partially in a projected portion defined by projecting said basket portion down toward said vibration plate.

3. An endoscope cleaner as defined in claim 1, wherein said basket portion is constituted by mesh material.

4. An endoscope cleaner as defined in claim 3, wherein said plural ultrasonic vibrators are arranged radially on circles which are concentric on a plane parallel with a surface of said vibration plate, and at least one of said ultrasonic vibrators is disposed under said basket portion and positioned on an innermost one of said circles.

5. An endoscope cleaner as defined in claim 3, wherein said plural ultrasonic vibrators are arranged in a spiral form starting from a point under said basket portion on a plane parallel with a surface of said vibration plate.

6. An endoscope cleaner as defined in claim 1, further comprising a disinfectant supply line for supplying said cleaning chamber with disinfectant to disinfect said endoscope and said small part.

7. An endoscope cleaner as defined in claim 6, further comprising:
a supply line for supplying said cleaning chamber with said cleaning fluid;
a drain port, formed in said cleaning chamber, for draining said cleaning fluid; and
a circulation line for circulating said cleaning fluid from said cleaning chamber through said nozzle opening and said drain port.

8. An endoscope cleaner as defined in claim 7, further comprising a water supply line for supplying said cleaning chamber with water after cleaning said endoscope and said small part and before disinfection, to rinse said cleaning chamber;
wherein said circulation line circulates said disinfectant after said disinfectant supply line operates, and circulates said water after said water supply line operates.

9. An endoscope cleaner as defined in claim 1, wherein said basket portion has a width increasing upwards.

10. An endoscope cleaner as defined in claim 1, wherein said small part is constituted by plural button parts or cap parts.

11. An endoscope cleaner as defined in claim 1, wherein said endoscope includes:
a handle portion;
an insertion tube disposed to extend from said handle portion;
a cable disposed to extend from said handle portion;
a peripheral portion of said cleaning chamber is assigned with said handle portion; and
said vibration plate supports said insertion tube and said cable upwards.

12. An endoscope cleaning method of cleaning an endoscope (10), comprising steps of:
placing said endoscope in a cleaning chamber (31) and a small part (21-23) thereof in a basket portion (53) which is disposed substantially at a center of said cleaning chamber;
supplying cleaning fluid (76) through at least one nozzle opening (47a) into said cleaning chamber for circulation cleaning of said endoscope and said small part, said nozzle opening being disposed under said basket portion; and
vibrating a vibration plate (32) disposed in a lower portion (31c) of said cleaning chamber by use of an ultrasonic vibrator (45) disposed under said basket portion at least partially.

13. An endoscope cleaning method as defined in claim 12, further comprising steps of:
after said cleaning step, rinsing said cleaning chamber with water; and
after said rinsing step, disinfecting said endoscope and said small part with disinfectant.

14. An endoscope cleaning method as defined in claim 13, further comprising steps of:
after said disinfecting step, rinsing said cleaning chamber with water; and
drying said endoscope and said small part with evaporative gas.

15. An endoscope cleaning method as defined in claim 13, wherein said circulation cleaning step includes:
draining said cleaning fluid from said cleaning chamber through a drain port; and
circulating said cleaning fluid from said cleaning chamber by use of said nozzle opening and said drain port.
